# EUROPEAN PATENT APPLICATION

(11) **EP 2 075 244 A1**
(43) Date of publication of application: **01.07.2009**
(21) Application number: 07025094.9
(22) Date of filing: 24.12.2007
(51) Int. Cl.: C07C 69/67, C07C 235/02, C07D 307/32

(54) **New route to building block for making renin inhibitors**

(71) Applicant: DSM IP Assets B.V., 6411 TE Heerlen (NL)
(72) Inventor: Mink, Daniel, 4700 Eupen (BE); Wolberg, Michael, 93073 Neutraubling (DE); De Vries, Andreas, Hendrikus, Maria, 6212 CR Maastricht (NL); Kaptein, Bernardus, 6133 BE Sittard (NL)
(74) Representative: Hoogendam, Gerrie Christine

(57) **Abstract**

Described is a method for the preparation of building blocks in the synthesis of renin inhibitors such as aliskiren. The method makes use of a oxirane compound of formula 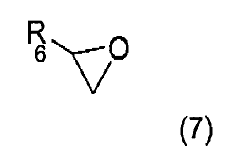 wherein R₆ stands for CH₂-halogen (F, Cl, Br, or I); C(O)Y, with Y being groups described in claim 1, or R₆ is CH₂-OR, wherein R stands for H or groups as described in claim 1.

## Description

### Field of the Invention

The invention pertains to a new route to building blocks useful in the convergent synthesis of certain 2(S),4(S),5(S);7(S)-2,7-dialkyl- 4-hydroxy-5-amino-8-aryl-octanoyl amide derivatives, or pharmaceutically acceptable salts thereof, such as the peptidomimetic compound aliskiren. The invention particularly relates to the use of of oxirane compounds for the preparation of stereospecific y-hydroxy-carbonyl compounds and/or its lactonized forms, en route to the convergent synthesis of said renin inhibitors, and to the reaction of the stereospecific Y-hydroxy-carbonyl compounds and/or the lactonized form thereof to the other main building block, viz. an organametallic reagent comprising the required structure for the aromatic part of these substituted octanoyl amide derivatives. Particularly, the 2(S),4(S),5(S),7(S)-2,7-dialkyl-4-hyclroxy-5-amino-8-aryl-octanoyl amide derivatives to which the methods of the present invention applies are any of those having renin inhibitory activity and, therefore, pharmaceutical utility, e.g., those disclosed in U.S. Patent No. 5,559,111, WO 2006/061427, or WO 2006/095020.

### Background of the Invention

The 2(S),4(S),5(S);7(S)-2,7-dialkyl- 4-hydroxy-5-amino-8-aryl-octanoyl amide derivatives, or pharmaceutically acceptable salts thereof to which the invention pertains, satisfy the general formula (1), 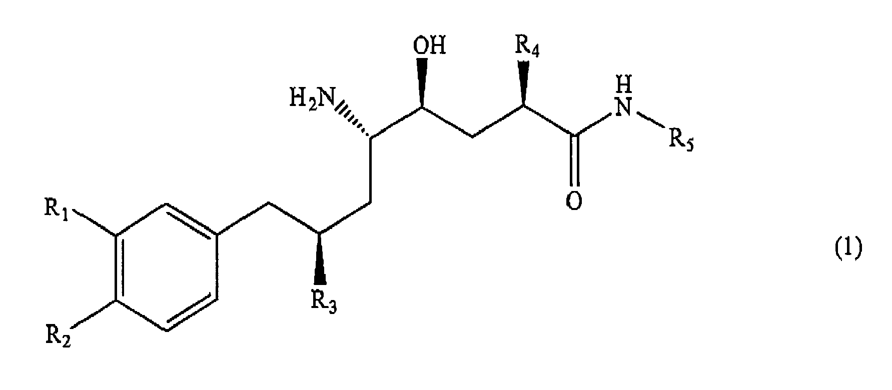 wherein R₁ is halogen, C₁₋₆halogenalkyl, C₁₋₆akoxy-C₁₋₆alkyloxy or C₁₋₆alkoxy-C₁₋₆alkyl; R₂ is halogen, C₁₋₆alkyl or C₁₋₆alkoxy; R₃ and R₄ are independently branched C₃₋₆alkyl; and R₅ being selected from the group consisting of C₁₋₁₂cycloalkyl, C₁₋₁₂alkyl, C₁₋₁₂hydroxyalkyl, C₁₋₆alkoxy-C₁₋₆alkyl, C₁₋₆alkanoyloxy-C₁₋₆alkyl, C₁₋₁₂aminoalkyl, C₁₋₆alkylamino-C₁₋₆alkyl, C₁₋₆dialkylamino-C₁₋₆alkyl, C₁₋₆alkanoylamino-C₁₋₆alkyl, HO-(O)C-C₁₋₁₂alkyl, C₁₋₆alkyl-O-(O)C-C₁₋₆alkyl, H₂N-C(O)-C₁₋₁₂alkyl, C₁₋₆alkyl-HN-C(O)-C₁₋₆alkyl, (C₁₋₆alkyl)₂-N-C(O)-C₁₋₆alkyl saturated, unsaturated, or partially saturated C₁₋₁₂heterocyclyl bonded via a carbon atom, and which heterocyclyl is optionally substituted one or more times by C₁₋₆alkyl, trifluoromethyl, nitro, amino, N mono- or *N,N-*di-C₁₋₆alkylated amino, C₁₋₆alkanoyl, C₂₋₆alkenyl, C₂₋₆alkynyl, C₁₋₆alkoxy, C₁₋₆alkoxycarbonylamino, C₀₋₆alkylcarbonylamino, C₁₋₆alkylcarbonyloxy, C₁₋₁₂aryl, *N*-mono or *N,N-*di-C₁₋₆alkylated carbamoyl, optionally esterified carboxyl, cyano, halogen, halo-C₁₋₆alkoxy, halo-C₁₋₆alkyl, C₁₋₁₂heteroaryl, saturated, unsaturated or partially saturated C₁₋₆heterocyclyl, hydroxyl, nitro; oxide or oxo which is bonded via a C atom;
or the salt of compound (1), especially a pharmaceutically acceptable salt thereof.

The above more particularly pertains to the synthesis of compounds of formula (3), the fumaric acid salt of which is known as aliskiren, (2S,4S,5S,7S)-7-(3-(3-methoxypropoxy)-4-methoxybenzyl)-5-amino-N-(2-carbamoyl-2-methylpropyl)-4-hydroxy-2-isopropyl-8-methylnonanamide.
In the present application, any reference to aliskiren is deemed to include reference to all pharmaceutically acceptable salts, and prodrugs thereof.

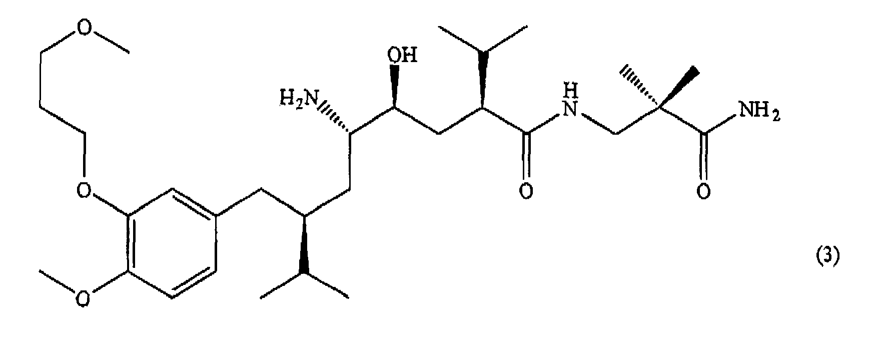

In the convergent synthesis of compound of formula (3), via a compound according to formula (2), it is known to employ a compound satisfying formula (4) as described by Sandham et al., Tetrahedron Letters 41 (2000) 10091-10094.

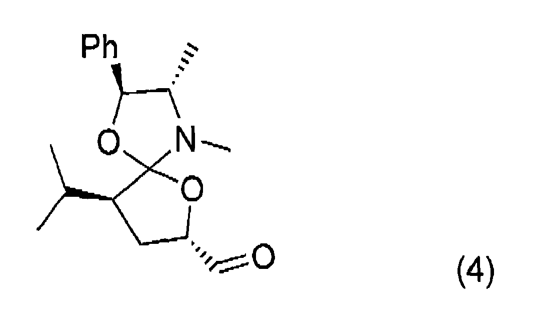

The compound according to formula (4) is reacted to a second compound, satisfying the structural formula (5a); 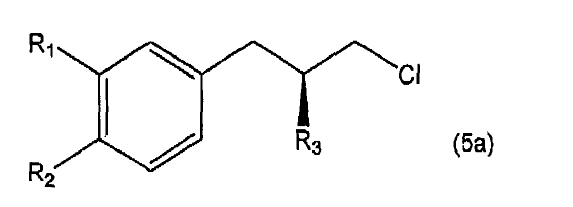 wherein compound of formula (5a) is first converted to the corresponding Grignard reagent, and subsequently transmetallated with CeCls as described by Sandham et al, and wherein R₁ is 3-methoxy-propoxy, R₂ is methoxy, and R₃ is 2-propyl, which reaction results, after work up, in the compound of formula (2), 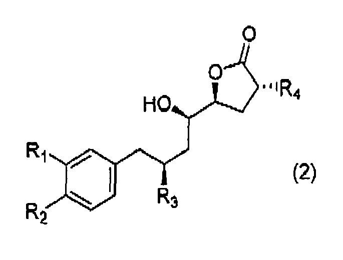 wherein R₁ is 3-methoxy-propoxy, R₂ is methoxy, and R₃ and R₄ are 2-propyl. The synthesis described by Sandham et al starts from a compound according to formula (6a), containing a chiral auxiliary which will not end up in the compounds of formula (2) or formula (3).

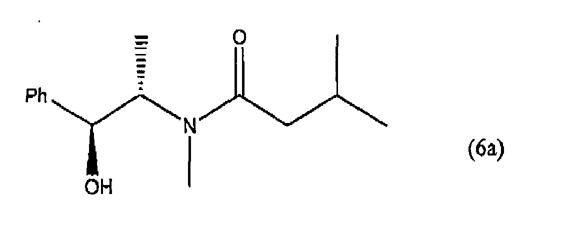

The synthesis of said compound of formula (4), starting from the compound of formula (6a) requires not fewer than seven reaction steps, with an overall yield of less than 25%, and also includes the use of a chiral auxiliary, in other words, using a stochiometric amount of a chiral group derived from a chiral compound, which is a relatively expensive group.

### The Invention

It would be advantageous to provide a route to compounds according to formula (10), suitable in the convergent synthesis of compounds of formula (1), and of fomula (3), which requires a reduced number of reaction steps, and/or which allows obtaining the compound, in the sterically desired configuration, with an improved yield. Further, it is an objective to provide a synthesis route in which one could elect to dispense with the use of an (expensive) chiral auxiliary. The invention aims at achieving one or more of the above objectives.

The invention now provides a novel synthesis route, based on the use of a oxirane compound of formula (7), according to the general reaction Scheme I.

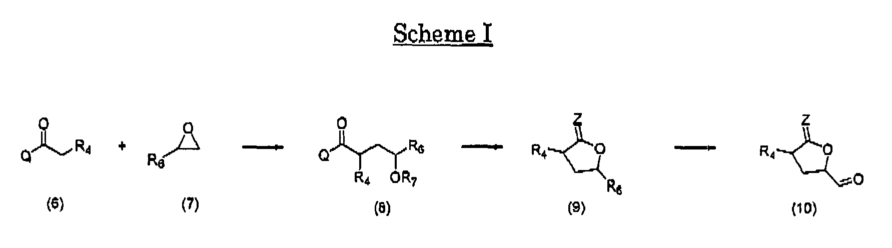

In one aspect of the invention, the compound according to formula (6) reacts with a oxirane compound of formula (7), which reaction results in the formation of a compound according to formula (8) and/or (9), and its subsequent conversion to the compound of formula (10),
wherein R₄ is branched C₃₋₆alkyl;
Q represents a C₁₋₁₂alkoxy, C₁₋₁₂aryloxy, C₁₋₁₂alkylaryloxy, C₁₋₁₂arylalkyloxy, OH, OC(0)C₁₋₁₂alkyl, OC(O)C₁₋₁₂alkylaryl, OC(O)C₁₋₁₂arylalk-yl, N(H)C₁₋₆alkyl, N(C₁₋₆alkyl)C₁₋₁₂alkyl; optionally substituted amino alcohols, such as 1-amino-2-ethanol, norephedrine and 1-amino-2-hydroxy-C₁₋₁₂alkyl, and 1-amino-2-hydroxy-C₁₋₁₂alkylaryl, and N-alkylated amino alcohols, such as N-methyl-lamino-2-ethanol, pseudoephedrine, ephedrine, and N-C₁₋₆alkyl-1-amino-2-hydroxy-C₁₋₁₂alkyl, and N-C₁₋₆alkyl-1-amino-2-hydroxy-C₁₋₁₂alkylaryl; R₆ is CH₂-halogen (F, Cl, Br, or I); C(O)Y, with Y being C₁₋₁₂alkoxy, C₁₋₁₂aryloxy, C₁₋₁₂alkylaryloxy, C₁₋₁₂arylalkyloxy, OH, H, Cl, OC(O)C₁₋₁₂alkyl, N(H)C₁₋₁₂alkyl, N(C₁₋₆alkyl)Cl₁₋₁₂alkyl, N(H)C(O)C₁₋₁₂alkyl, N(C₁₋₆alkyl)C(O)C₁₋₁₂alkyl; or R₆ is CH₂-OR, wherein R stands for H, C₁₋₁₂alkyl, C₁₋₆alkyl-C₁-₆alkoxy, C₁₋₆alkoxy-C₁₋₆alkyl, or C₁₋₁₂alkylaryl, C₁₋₁₂arylakyl, C(O)C₁₋₁₂alkyl, C(O)C₁₋₁₂alkylaryl, C(O)C₁₋₁₂arylalkyl, C(O)OC₁₋₆alkyl, C(O)N(H)C₁₋₆alkyl C(O)N(C₁₋₆alkyl)C₁₋₁₂alkyl;
R₇ represents H, or is an O-protecting group as decribed in J. F. W. McOmie, "Protective Groups in Organic Chemistry", Plenum Press, London and New York 1973; or in T. W. Greene and P. G. M. Wuts, "Protective Groups in Organic Synthesis", Third edition, Wiley, New York 1999;
Z is oxygen or a "masked oxygen atom". For the purpose of this invention, a "masked oxygen" is defined as the five membered ring containing an O and N atom depicted in formula (9a).

In another aspect of the invention, the group Q in compound of formula (6) is a chiral group in optical pure form, such as pseudoephedrine in compound of formula (6a), or the oxirane compound of formula (7) is optically pure, or both items are optically pure, the compounds according to formula (8), (9), and (10) can be obtained in optically enriched form, preferably in optically pure form of the desired configuration matching with the 2(S), and 4(S) center of compounds according to formula (1), (2), and (3).
As an example a sequence of reactions using an optically pure oxirane compound is depicted in Scheme II.

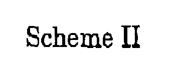

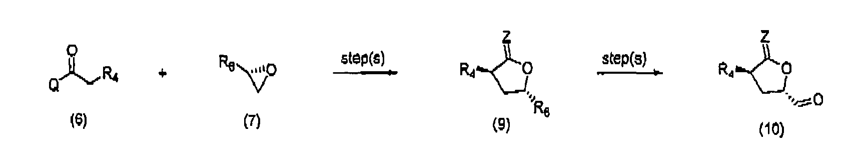

The invention also relates to the following compounds:
a compound satisfying formula (6): 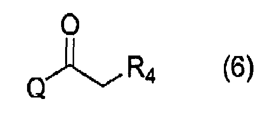
an oxirane compound that satisfies formula (7) 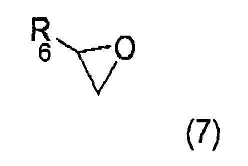
compounds according to formula (8) and/or the lactonized form thereof, i.e compounds according to formula (9), 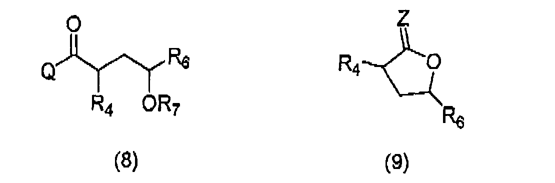
and compounds according to formula (10), 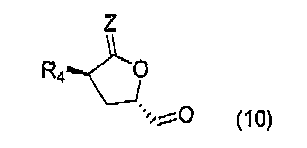
wherein R₄, R₆, R₇, Q, and Z have the meaning as described above.

If the group Q in the compound of formula (6) is an 1-amino-2-hydroxyC₁-₁₂alkane (chiral or non chiral), such as the pseudoephedrine moiety, or 1-amino-2-ethanol, optionally alkylated on the amine, the result for group Z in formula (9) can be a five membered ring, described above as a "masked oxygen", such as the upper ring depicted in Formula 9a: 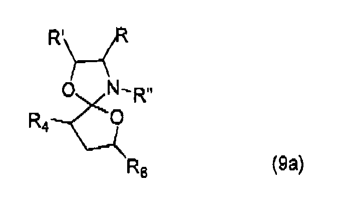 wherein R, R', and R" each independently can be H, C₁₋₁₂alkyl, C₁₋₆alkyl-C₁₋₆alkoxy, C₁₋₆alkoxy-C₁₋₆alkyl, C₁₋₁₂alkylaryl, or C₁₋₁₂arylalkyl; or R, with R', or R with R", or R' with R" can form an optionally substituted (heteroatom containing) ring. Optionally substituted groups are all kind of groups which will not interfere with the aimed reaction and/or reactions afterwards.

The invention further pertains to the reaction of the above depicted compound of formula (4), preferably prepared according to the general schemes I and II, and the compound of formula (5), 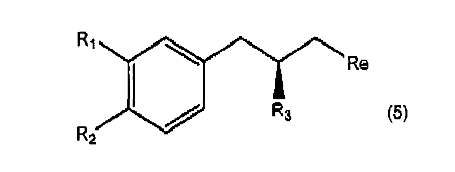 wherein R₁, R₂, and R₃ have the above mentioned meaning, and Re denotes a reactive moiety selected from F; Cl; Br; I; M(X)ₙ, wherein X is F, Cl, Br, I, CN, C₁₋₁₂alkyl, C₁₋₆alkoxy and M is a metal, preferably M is Mg, Ce, Li, Ba, Al, B, Cu, Zn, Mn, Ti, Zr, In, and n is 0, 1, 2, 3, or 4; MM'(X)ₙ(Y)_{n'}, wherein M and M' are a metal, preferably M and M' are each independently Mg, Ce, Li, Ba, Al, B, Cu, Zn, Mn, Ti, Zr, In, and X and Y are each independently chosen from F, Cl, Br, I, or CN, C₁₋₁₂alkyl, C₁₋₆alkoxy; n and n' are each independently chosen from the values as described above for n;
optionally in the presence of a metal complex, preferably a metal complex used in catalytical amounts, and optionally in the presence of an additive,
which reaction results in the formation of compound of formula (2).
Said metal complex can be any metal complex, preferably transition metal complexes, more preferred metal complexes derived from group VII and group VIII of the periodic system, most preferred manganese or iron complexes are used, such as MnCl₂, and FeCl₃, Fe(acac)₃, FeCl₂.
Suitable additives are Lewis acids, such as ZnCl₂, CuCl; CuI, InCl₃, TiCl₄, alkali metal salts, such as LiCl; tertiary amines and tertiary diamines, such as Et₃N, N-methylpyrrolidine, tetramethylendiamine (TMEDA); amides and ureas, such as *N-*methylpyrrolidinone (NMP) and 1,3-dimethyl-2-oxohexahydropyrimidine (DMPU); hexamethylphosphoric acid triamide (HMPA) ; tris(dialkylamino)phosphines, such as tris(dimethylamino)phosphine (HMPT).
Said metal complexes or additives can be chiral to enhance the amount of compound according to formula (2), with the desired configuration at the C-5 stereogenic center. Chiral metal complexes can be prepared beforehand or prepared in situ, by mixing the metal complex with an suitable chiral ligand. Suitable chiral ligands are known to the person skilled in the art, for example chiral amino alcohols, optionally alkylated at the amine functionality, such as ephedrine; chiral phosphor containing compounds, such as (3,5-dioxa-4-phosphacyclohepta[2,1-a;3,4-a']dinapthalen-4-yl)dimethylamine (MonoPhos), chiral bisphosphines, such as 2,2'-Bis(di-*p*-tolylphosphino)-1,1'-binaphthyl (BINAP). Preferably ,the chiral metal complex is used in catalytical amounts. Suitable chiral additives are any additive mentioned above with a stereogenic center or other form of chirality. Preferred are chiral amines and amine derivatives, such as *N,N*-dimethylamino acid methyl esters, *N,N-*dimethylmethylbenzylamine and chiral phosphor containing compounds, such as (3,5-dioxa-4-phosphacyclohepta[2,1-a;3,4-a']dinapthalen-4-yl)dimethylamine (MonoPhos).

In yet another aspect the invention provides the reaction of compound of formula (8) and/or its lactonized compound of formula (9), preferably with R₆ being C(O)H, with the compound of formula (5), optionally in the presence of a metal complex and/or an additive, as described above for the coupling of compounds of formula (4) and formula (5),
which reaction results in the formation of compound according to formula (2a) and/or its lactonized compound according to formula (2), 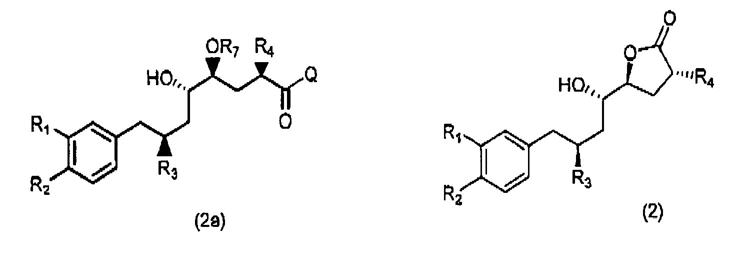 wherein R₁, R₂, R₃, R₄, and R₇ have the above given meaning.

### Further description of the invention

Surprisingly, it has now been found that the reaction of oxiranes according to formula (7) with compounds of formula (6), results in the formation of a compound according to formula (8) and/or the formation of its lactonized form according to formula (9). Depending on the group Q and the conditions applied, there is little to none or a lot of compound according to formula (9) present. If the group Q in the compound of formula (6) is an optionally N-alkylated 1-amino-2-hydroxy alkyl (chiral or non chiral, the result for group Z in the general formula (9) can be the five membered ring as described above ("masked oxygen"), as described in (9a), or the chiral auxiliary will be hydrolysed resulting in the compound (9) wherein Z is oxygen, depending on the conditions used during the reaction and/or work-up. Under strong acidic conditions the double bonded oxygen is preferred. More details, and suitable conditions to apply are also described by S.K. Taylor, in Tetrahedron 56 (2000), 1149-1163, equation 38, and references therein

The preparation of the lactone and the masked equivalent, here as an example now shown with R₄ is 2-propyl, and being all part of the invention, are exemplified in Scheme III.

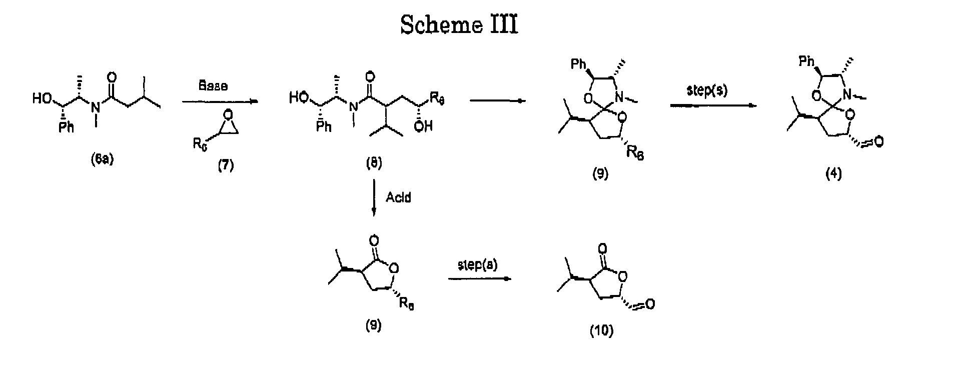

The reaction of a compound similar to formula (6a) with oxiranes of formula (7) have been described by S. K. Taylor, in Tetrahedron 56 (2000), 1149-1163, and references therein. Suitable conditions and bases for these reactions are given by Taylor and/or in the cited references. Suitable bases are for example lithium based bases, for example LDA, and LiHMDS. Optionally the enolates formed by reacting compounds of formula (6) with a suitable base, are first transmetallated to the corresponding aluminium enolate, for example with Et₂AlCl, before reacting with compounds of formula (7).
The use of this type of reactions for compound (6) and oxiranes of formula (7) for the preparation of compounds of formula (8), (9), (4) and (10), and compounds further in the route to compounds of formula (1) is new.

Surprisingly, the reaction of epichlorohydrin as a compound of formula (7) with the compound according to formula (6a) results in the formation of compounds of formula (8) and/or (9) and after further reaction steps in compounds of formula (4) and (10). More preferred the use of S-epichlorohydrin results in the formation of compounds of formula (4), (8), (9) and (10) with the desired (S) configuration of the C-2 and C-4 sterogenic centers, as is exemplified in Scheme IV.

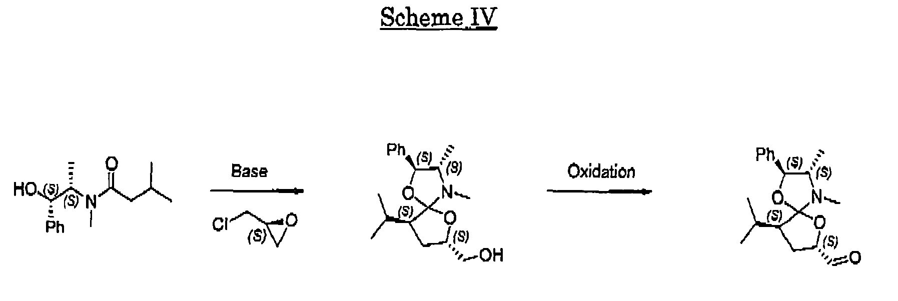

Suitable bases for this transformation are the same as given by Taylor in the refence mentioned above. The amount of base in our applications is preferably between 0.8 and 3 equivalents, also depending on the amount of acidic protons present in the compound of formula (6). In the examples of scheme III and IV, with the free alcohol present, preferably at least 1.8 equivalents of base are used to obtain the highest yield.
The oxidation of the alcohol group to the aldehyde group, as for example depicted in Scheme IV, can be performed by any known oxidation method, for example analogue to Sandham et al., Tetrahedron Letters 41 (2000) 10091-10094, or other methods using stoichiometric amounts of oxidant. Catalytic oxidation using a relative clean and cheap oxidant are also possible. Such catalytic systems are for example described in Tetrahedron 59, 2003, 6739-6750; Advanced Synthesis & Catalysis, 2005, 347; 1423-1434; Journal of Molecular Catalysis B: Enzymatic 39, 2006, 3-8; Organic Letters, 2005, 7, 4879-4882; Angewandte Chemie, Int. Ed. 2007, 46, 3567-3570; and J. Org. Chem. 2005, 70, 3343-3352, and references herein. Preferably catalytic oxidation methods are used.

Scheme V shows a reaction of a non chiral compound of formula (6), for example an ester, with optically pure (S)-epichlorohydrin

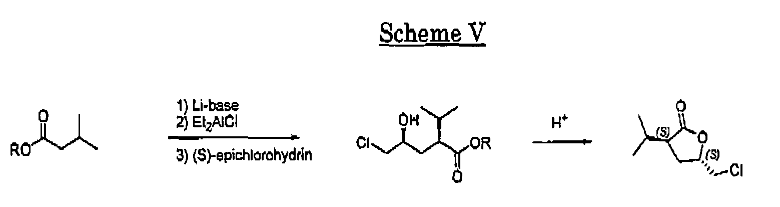

The reaction of similar enolates with oxiranes, but not with epichlorohydrin, and which conditions and bases to apply, are described by S. K. Taylor, in Tetrahedron 56 (2000), 1149-1163, and references therein.
In this aspect of the invention, the oxirane is (S)-epichlorohydrin, and the compound according to formula (6) is preferably an ester as depicted in scheme IV. More preferable compound of formula (6) is a tertiar butyl ester (R is tert-butyl). Surprisingly, as is shown in scheme IV, also without the aid of a chiral auxiliary in compound of general formula (6), the optical pure oxirane reactant of the invention allows for obtaining the required stereochemistry in compounds of formula (8) and (9), and subsequently (10),

Optionally the resulting compound of Scheme V, and compounds obtained using related compounds of general formula (6) and (7), can be converted by known methods to the corresponding aldehyde compound of the general formula (10). Such methods could comprise:
1) the replacement of the chloride by an acetate using for example a (C₁₋₈-alkyl)₄N acetate salt in stoichiometric amounts or, optionally a catalytic amount of a (C₁₋₈-alkyl)₄N acetate salt in the presence of any cheap and readily avaible acetate salt, such as potassium acetate or sodium acetate.
2) the oxidation of the obtained alcohol to the aldehyde using methods known in the art, and as described above.

Optionally, the resulting compound of scheme V, and compounds obtained using related compounds of general formula (6) and (7), can also be converted to compound according to formula (9), and subsequently to compound of formula (10) as depicted in Scheme VI.

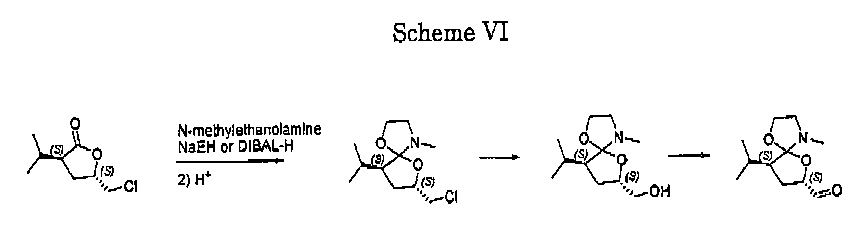

The ring-opening aminolysis of lactones in general, leading to amides are known, and for example described in Tetrahedron Letters 42 (2001), 2439-2441, and Tetrahedron Letters 42 (2001), 9039-9041. The subsequent formation of the five membered ring ("masked oxygen") will go analogues to as described above, and also as described in Sandham et al., Tetrahedron Letters 41 (2000) 10091-10094..

The order of reactions as described above can also be reversed, hence first the formation of the amide, followed by reaction with the appropriate oxirane. An example is depicted in Scheme VII.

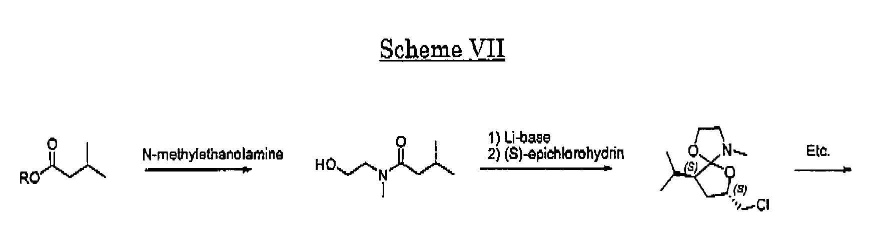

The aminolysis of esters in general, to amides is known, and for example described in Tetrahedron Letters 42 (2001), 9039-9041.

If the oxirane compound is a glycidate ester, the reaction sequences as depicted in scheme VIII are part of the invention resulting in compound according to formula (9) with R₆ being an ester group: 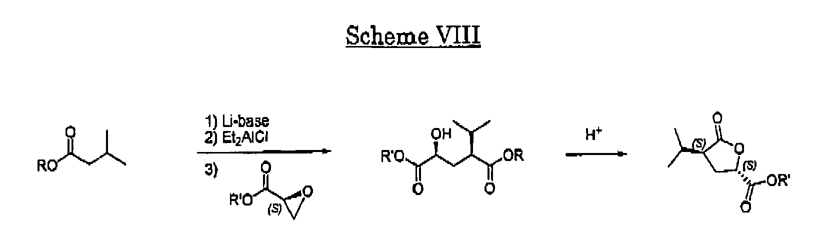 wherein R and R' are chosen from C₁₋₁₂ alkyl, C₁₋₁₂ alkylaryl, C₁₋₁₂ arylalakyl, preferably R and R' are C₁₋₆alkyl, and more preferably R is tertiar-butyl, and R' is methyl or ethyl.
Optionally, the resulting compound of Scheme VIII can be converted to the corresponding aldehyde compound of Formula (10) by methods known in the art to convert a carboxy ester to the corresponding aldehyde, and optionally by first the formation of the five membered ring analogues as described above ("masked oxygen"). In this sense the 5-membered ring is a protecting group of the lactone moiety.

With the oxirane compound being a glycidol based ether or ester, a possible way to synthesize a compound according to formula (9) is depicted in Scheme IX:

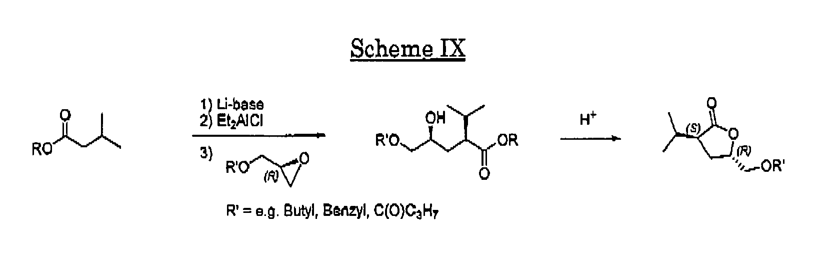

Optionally, the resulting compound of Scheme IX can be converted to the corresponding aldehyde compound of formula (10) by methods known in the art, and optionally by first the formation of the 5-membered ring ("masked oxygen") analogues as described above. In this sense the 5-membered ring is a protecting group of the lactone moiety,

As also described above this sequnece of reactions can be perfomed in reversed order, see scheme X:

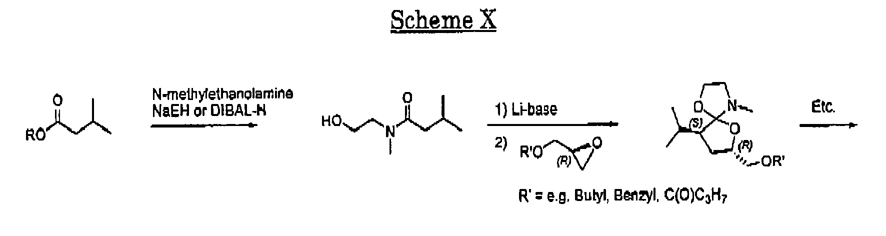

The N-methyl group in the Q-moiety of the reactant according to formula (6) can also be introduced in a separate reaction, or a subsequent stage. This is illustrated with reference to an exemplary Scheme X. The methylation will occur in a manner known in the art, e.g. using MeI, or Me₂SO₄

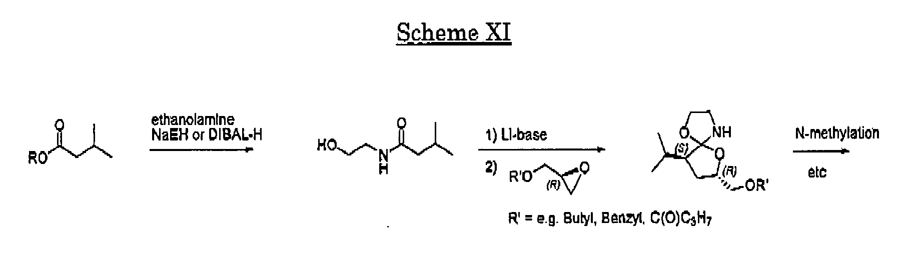

Since more acidic protons are present in this aspect of the invention, more equivalents of bases will be needed. In the above depicted sequence in Scheme XI, at least 2,7 equivalents of base will be used to obtain a high yield of desired compound.

Reaction sequences as depicted in the several schemes above where the smallest amount of base can be used to obtain high yields are preferred.

In all reactions and schemes with the oxirane reactant present, optionally any Lewis acid can be added to facilitate the reaction. Suitable Lewis acids are for example BF₃ etherate, AlCl₃, CuCl, etc.

In all the above schemes and examples it should be noted that wherever compound of Formula (6) is used the corresponding malonate as depicted in Scheme XII can also be used.
Subsequent reactions should comprise somewhere in the sequence a hydrolysis and decarboxylation step leading to the same compounds as Formula (8), (9), and (10). For clarity the hydrolyis and decarboxylation is here performed straight after the reaction with the oxirane, but can be performed at any stage of the sequence.

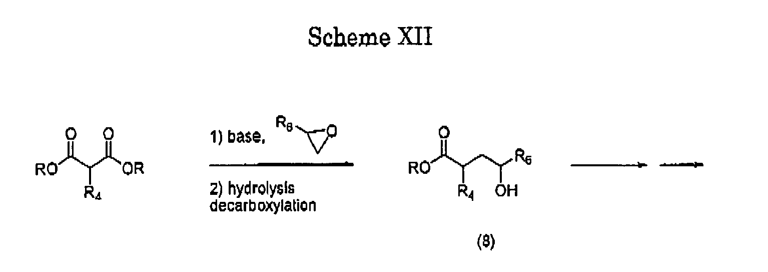

The compound of formula (9) can also be obtained through a route in which the reaction product of compound of formula (6) and a suitable allyl reagent is obtained in optically pure from by any well known resolution method, hence not using a chiral group Q. A suitable sequence of reactions is depicted in Scheme XIII, 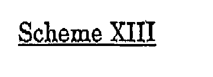 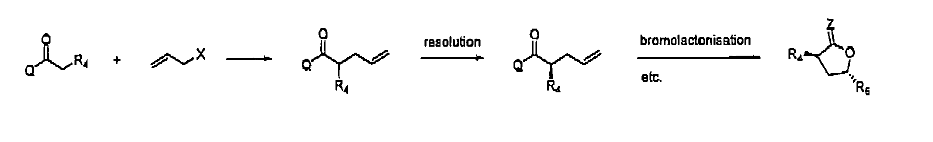 wherein R₄ and R₆ are as described above, and Q is as described above, preferably Q is a non-chiral variant thereof, more preferably Q is C₁₋₄-alkoxy, OH, or aminoethanol;
and X is a leaving group. Leaving groups are known in the art, suitable leaving groups are for example -OC(O)CH₃, -OC(O)CF₃, -OSO₂CF₃, -OSO₂CH₃, - OSO₂CH₃C₆H₄, -OC(O)OCH₃, or -QC(O)OC₄H₉.
Suitable resolution methods are classical salt formation, a separation using a chiral stationary phase, for example the so-called simulating moving beds, or an enzymatical resolution using for example a suitable lipase. Preferred are enzymatical resolution methods. More preferred is the use of pig-liver esterase or mutants thereof.
The undesired enantiomer can be recovered and racemised using racemising techniques. Such racemising techniques for carbon centers alpha to a carbonyl moiety are known in the art, for example stirring with base, optionally at elevated temperatures.
Routes to compound of formula (9) according to Scheme XIII have the advantage that stoichiometric amounts of expensive chiral auxiliaries can be avoided.
The bromo-lactonisation and subsequent reactions can be performed analogues to Sandham et al., Tetrahedron Letters 41 (2000) 10091-10094.

### Overall Synthesis

The invention also related to the use of the compounds resulting from the foregoing synthesis steps, in a convergent synthesis to compounds of formula (1), (2), or (3).

To this end, compounds of formula (10) are coupled to a building block for the aromatic part of the desired structure, represented by the foregoing formula (5).
The compound of formula (5a) can be made in known ways. References in this respect are Sandham et al. Tetrahedron Lett. 41, (2000) 10091-10094 and Sturm et al. Adv. Synth, Catal. 2003, 345, 160-164.

The only example describing the preparation of compound according to formula (2) by reacting a compound of formula (5a) with another suitable coupling partner is described by Sandham et al. Tetrahedron Lett. 41, (2000) 10091-10094, and comprises first preparing the Grignard compound of formula (5a), followed by transmetallation with CeCl₃, prior to the addition to the compound according to formula (4). The resulting compound of formula (2) is obtained in 51% isolated yield and 85:15 ratio of diastereomers. Ratio of diastereomers could be improved by the addition of tetramethyl ethylenediamine (TMEDA) to 96:4, albeit at the expensive of the yield: 33%.

The invention also relates to improvements with respect to this coupling reaction, i.e reacting compound (5) with a compound of formula (10).
These improvements are described above, and are preferably by reacting the compounds of formula (5) and (10) in the presence of a metal complex and/or additive, for example:
- the use of an other organometallic reagents instead of the Grignard, e.g. lithium, zinc, copper, zirconium, titanium, manganese, and iron.
- the addition of lithium chloride to any of the organometallic reagents, including the Grignard and Cerium compound;
- the addition of a catalyst in the coupling reaction of any of the organometallic reagents. Such catalysts can be any transition metal catalyst, or a Lewis acid catalyst, e.g. ZnCl₂, or BF₃-etherate
- the addition of an agent to improve diastereoselectivity. Suitable agents for this are DMPU (1,3-Dimethyl-3,4,5,6-tetrahydro-2(1H)-pyrimidinone) and HMPT (hexamethyl phosphor triamide);
- any combination, or multiple combination of the above. For example the use of the Grignard compound of formula (5), with the addition of LiCl (preferably catalytically), and the addition of HMPA (preferably catalytically). Other suitable combination could be the use of the Manganese organometallic reagent and the presence of a suitable Lewis acid, e.g. BFs-etherate.

Suitable solvents for the coupling of (10) and (5) are polar solvents, preferably non-protic. Preferred are solvents showing good solubility profiles for these kind of compounds, for example ethers and cyclic ethers, dioxane, toluene, and any mixture thereof.
The temperature is not critical, and is depending on the organometallic reagent, the solvent and extra reagents used. Typically, the temperature is chosen to lie between -100 C° and + 100°C.
The further processing into compounds of formula (1), and preferably of aliskiren, follows readily from the art. Reference is made e.g. to the aforementioned Sandham et al. Tetrahedron Lett. 41, (2000) 10091-10094.

### Salts

As mentioned above in respect of aliskiren, the compounds of formula (1) include salts, especially pharmaceutically acceptable salts. Salts and pharmaceutically acceptable salts of the compound according to formula 910 have been described in US patent 5,559,111, column 11, line 50 to column 12, line 35, and are incorporated by refernce herein. In view of the close relationship between the compounds and intermediates in free form and in the form of their salts, including those salts that can be used as intermediates, for example in the purification or identification of the compounds or salts thereof, any reference to "compounds", "precursors" and "intermediates" is to be understood as referring also to one or more salts thereof or a mixture of a corresponding free compound, intermediate or starting material and one or more salts thereof each of which is intended to include also any solvate, metabolic precursor such as ester or amide of the compound of formula (1), or salt of any one or more of these, as appropriate and expedient and if not explicitly mentioned otherwise. Different crystal forms may be obtainable and then are also included.

### General Process Conditions

The following, in accordance with the knowledge of a person skilled in the art about possible limitations in the case of single reactions, applies in general to all processes mentioned in the foregoing description, or hereinafter in the Examples and Claims, while reaction conditions specifically mentioned above or below are preferred:
All the above-mentioned process steps can be carried out under reaction conditions that are known per se, preferably those mentioned specifically, in the absence or, customarily, in the presence of solvents or diluents, preferably solvents or diluents that are inert towards the reagents used and dissolve them, in the absence or presence of catalysts, condensation or neutralizing agents, for example ion exchangers, such as cation exchangers, e.g. in the H⁺ form, depending on the nature of the reaction and/or of the reactants at reduced, normal or elevated temperature, for example in a temperature range of from about -100°C to about 190°C, preferably from approximately -80°C to approximately 150°C, for example at from -80 to -60°C, at room temperature, at from -20 to 40 °C or at reflux temperature, under atmospheric pressure or in a closed vessel, where appropriate under pressure, and/or in an inert atmosphere, for example under an argon or nitrogen atmosphere.

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofurane or dioxane, liquid aromatic hydrocarbons, such as benzene or toluene, alcohols, such as methanol, ethanol or 1- or 2-propanol, nitriles, such as acetonitrile, halogenated hydrocarbons, e.g. as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydrides, such as lower alkanoic acid anhydrides, for example acetic anhydride, cyclic, linear or branched hydrocarbons, such as cyclohexane, hexane or isopen- tane, or mixtures of these, for example aqueous solutions, unless otherwise indicated in the description of the processes. Such solvent mixtures may also be used in working up, for example by chromatography or partitioning. Where required or desired, water-free or absolute solvents can be used.

Where required, the working-up of reaction mixtures, especially in order to isolate desired compounds or intermediates, follows customary procedures and steps, e.g. selected from the group comprising but not limited to extraction, neutralization, crystallization, chromatography, evaporation, drying, filtration, centrifugation and the like. The invention relates also to those forms of the process in which a compound obtainable as intermediate at any stage of the process is used as starting material and the remaining process steps are carried out, or in which a starting material is formed under the reaction conditions or is used in the form of a derivative, for example in protected form or in the form of a salt, or a compound obtainable by the process according to the invention is produced under the process conditions and processed further in situ. In the process of the present invention those starting materials are preferably used which result in compounds of formula (1) which are described as being preferred. The invention relates also to novel starting compounds described herein, especially those leading to compounds mentioned as preferred herein.

It is to be understood that the invention is described in the claims and not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

## Claims

**1.** A method for the preparation of a compound of formula (8) and/or its lactonized form according to formula (9), 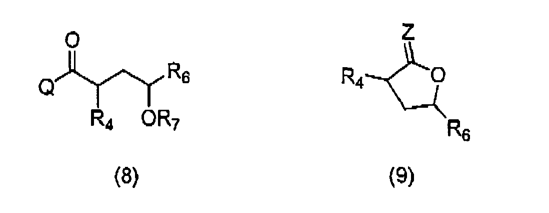 wherein R₄ is branched C₃₋₆alkyl; R₆ is CH₂-halogen (F, Cl, Br, or I); C(O)Y, with Y being C₁₋₁₂alkoxy, C₁₋₁₂aryloxy, C₁₋₁₂alkylaryloxy, C₁₋₁₂arylalkyloxy, OH, H, Cl, OC(O)C₁₋₁₂alkyl, N(H)C₁₋₁₂alkyl, N(C₁₋₆alkyl)C₁₋₁₂alkyl, N(H)C(O)C₁₋₁₂alkyl, N(C₁₋₆alkyl)C(O)C₁₋₁₂alkyl; or R₆ is CH₂-OR, wherein R stands for H, O₁₋₁₂alkyl, C₁₋₆alkyl-C₁₋₆alkoxy, C₁₋₆alkoxy-C₁₋₆alkyl, or C₁₋₁₂alkylaryl, C₁₋₁₂arylalkyl, C(O)C₁₋₁₂alkyl, C(O)C₁₋₁₂alkylaryl, C(O)C₁₋₁₂arylalkyl, C(O)OC₁₋₆alkyl, C(O)N(H)C₁₋₆alkyl C(O)N(C₁₋₆akyl)C₁₋₁₂alkyl; R₇ represents H, or is an O-protecting group and Z is oxygen or a "masked oxygen atom", and wherein Q is as described below for compound (6),
the method comprising reacting a compound of formula (6) 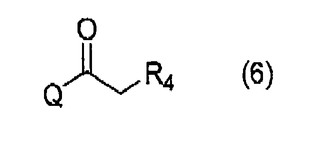 wherein Q represents a C₁₋₁₂alkoxy, C₁₋₁₂aryloxy, C₁₋₁₂alkylaryloxy, C₁-₁₂arylalkyloxy, OH, OC(O)C₁₋₁₂alkyl, OC(O)C₁₋₁₂alkylaryl, OC(O)C₁₋₁₂arylalkyl, N(H)C₁₋₆alkyl, N(C₁₋₆alkyl)C₁₋₁₂alkyl; optionally substituted amino alcohols, such as 1-amino-2-ethanol, norephedrine and 1-amino-2-hydroxy-C₁₋₁₂alkyl, and 1-amino-2-hydroxy-C₁₋₁₂alkylaryl, and N-alkylated amino alcohols, such as N-methyl-lamino-2-ethanol, ephedrine, and N-C₁₋₆alkyl-1-amino-2-hydroxy-C₁₋₁₂alkyl, and N-C₁₋₆alkyl-1-amino-2-hydroxy-C₁₋₁₂alkylaryl with an oxirane compound satisfying formula (7) 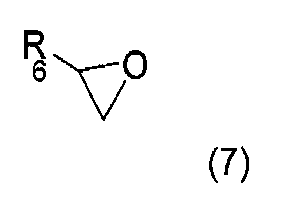 wherein R₆has the meaning given above,
in the presence of a suitable base.

**2.** A method according to claim 1, wherein the oxirane compound is epichlorohydrin.

**3.** A method according to claim 1 or 2, wherein the group Q in the compound of formula (6) is an optionally N-alkylated 1-amino-2-hydroxyC₁₋₁₂alkyl, the result for group Z in formula (9) being a five membered ring containing an N-atom and an O-atom, as depicted for the compound according to formula 9a: 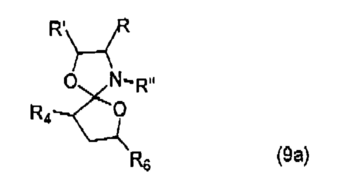 wherein R, R', and R" each independently can be H, C₁₋₁₂alkyl, C₁₋₆alkyl-C₁₋₆alkoxy, C₁₋₆alkoxy-C₁₋₆alkyl, C₁₋₁₂alkylaryl, or C₁₋₁₂arylalkyl; or R, with R', or R with R", or R' with R" can form an optionally substituted (heteroatom containing) ring.

**4.** A method according to claim 3, wherein the optionally N-alkylated 1-amino-2-hydroxyC₁₋₁₂alkyl group is chiral, preferably chiral groups derived from ephedrine, norephedrine or pseudoephedrine.

**5.** A method according to any one of the preceding claims, wherein a compound of formula (8) 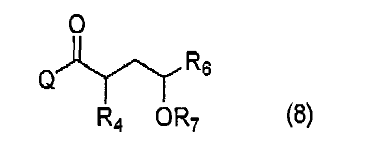 is formed which is lactonized so as to form the compound of formula (9).

**6.** A method according to claim 5,wherein a compound according to formula (8) is subjected to acidic conditions so as to form the compound of formula (9).

**7.** A method for the preparation of a compound of formula (2a), and/or the lactonized form thereof, i.e the compound according to formula (2) 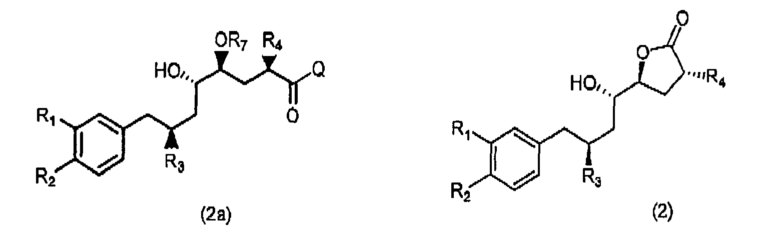 wherein R₁ is halogen, C₁₋₆halogenalkyl, C₁₋₆alkoxy-C₁₋₆alkyloxy or C₁₋₆alkoxy-C₁₋₆alkyl; R₂ is halogen, C₁₋₆alkyl or C₁₋₆alkyoxy; R₃ and R₄ are independently branched C₃₋₆alkyl, comprising reacting a compound satisfying formula (8) and/or its lactonized form according to formula (9) as defined above, and R₆ being preferably C(O)H (aldehyde), with a second compound satisfying formula (5) 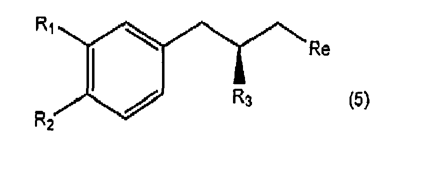 wherein R₁, R₂, and R₃ have the above mentioned meaning, and Re denotes a reactive moiety selected from F; Cl; Br; I; M(X)ₙ, wherein X is F, Cl, Br, I, CN, C₁₋₁₂alkyl, C₁₋₆alkoxy and M is a metal, preferably M is Mg, Ce, Li, Ba, Al, B, Cu, Zn, Mn, Ti, Zr, In, and n is 0, 1, 2, 3, or 4; MM'(X)ₙ(Y)_{n'}, wherein M and M' are a metal, preferably M and M' are each independently Mg, Ce, Li, Ba, Al, B, Cu, Zn, Mn, Ti, Zr, In, and X and Y are each independently chosen from F, Cl, Br, I, or CN, C₁₋₁₂alkyl, C₁₋₆alkoxy; n and n' are each independently chosen from the values as described above for n; optionally in the presence of a suitable catalyst and/or a suitable additive,
said compounds according to formula (8) or (9) being obtainable by a method according to any one of the preceding claims.

**8.** A method according to claim 7, wherein Re of the compound according to formula (5) is a M(X)n with M being Mg, Ce, Li, Mn or Zn, X and n being as described in claim 7, and wherein the reaction is conducted in the presence of a suitable iron catalyst, chosen from LiCl, a Lewis acid, a tertiary amine or tertiary diamine, an amide, an urea, or in the presence of a mixture of said catalyst and/or said additives.

**9.** A method according to any one of the claims 7 and 8, wherein the reaction is conducted in the presence of an additive to improve diastereoselectivity, preferably DMPU (1,3-Dimethyl-3,4,5,6-tetrahydro-2(1H)-pyrimidinone) or HMPT (hexamethyl phosphor triamide).

**10.** A method for the preparation of a compound of formula (8) and/or its lactonized form according to formula (9), 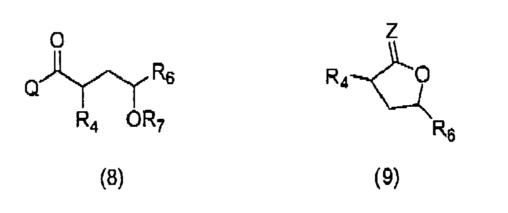 the method comprising reacting a compound of formula (6) 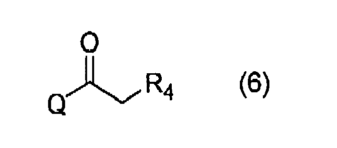 wherein R₄, R₆, R₇, and Z have the meaning as given in claim 1, and Q is any non-chiral version of the descriptions given in claim 1,
with an allyl reactant X-CH₂-CH=CH₂, wherein X is a leaving group

**11.** The use of a oxirane compound of formula (7) 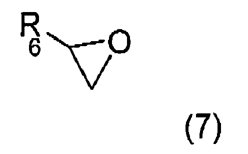 as defined in claim 1, as a reactant in the synthesis of a compound of formula (1) 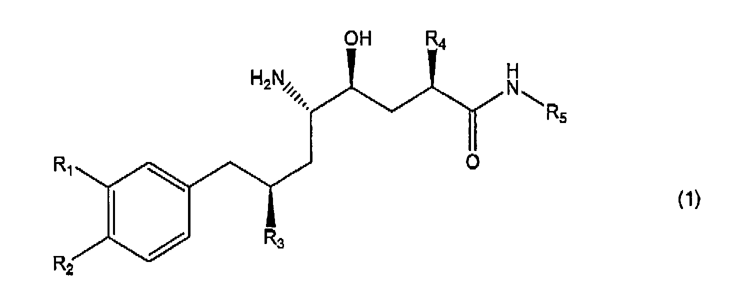 wherein R₁ is halogen, C₁₋₆halogenalkyl, C₁₋₆alkoxy-C₁₋₆alkyloxy or C₁₋₆alkoxy-C₁₋₆alkyl; R₂ is halogen, C₁₋₆alkyl or C₁₋₆alkoxy; R₃ and R₄ are independently branched C₃₋₆alkyl and R₅ is C₁₋₁₂cycloalkyl, C₁₋₁₂alkyl, C₁₋₁₂hyclroxyalkyl, C₁₋₆alkoxy-C₁₋₆alkyl, C₁₋₆alkanoyloxy-C₁₋₆alkyl, C₁₋₁₂aminoalkyl, C₁₋₆alkylamino-C₁₋₆alkyl, C₁₋₆dialkylamino-C₁₋₆alkyl, C₁₋₆alkanoylamino-C₁₋₆alkyl, HO-(O)C-C₁₋₁₂alkyl, C₁₋₆alkyl-Q-(O)C-C₁₋₆alkyl, H₂N-C(O)-C₁₋₁₂alkyl, C₁₋₆alkyl-HN-C(O)-C₁₋₆alkyl, (C₁₋₆alkyl)₂-N-C(O)-C₁₋₆alkyl; saturated, unsaturated, or partially saturated C₁₋₁₂heterocyclyl bonded via a carbon atom, and which heterocyclyl is optionally substituted one or more times by C₁₋₆alkyl, trifluoromethyl, nitro, amino, *N-*mono- or *N,N-*di-C₁₋₆lkylated amino, C₁₋₆alkanoyl, C₂₋₆alkenyl, C₂₋₆alkynyl, C₁₋₆alkoxy, C₁₋₆alkoxycarbonylamino, C₀₋₆alkylcarbonylamino, C₁₋₆alkylcarbonyloxy, C₁₋₁₂aryl, *N-*mono or *N,N-*di-C₁₋₆alkylated carbamoyl, optionally esterified carboxyl, cyano, halogen, halo-C₁₋₆alkoxy, halo-C₁₋₆alkyl, C₁₋₁₂heteroaryl, saturated, unsaturated or partially saturated C₁₋₆heterocyclyl hydroxyl, nitro; oxide or oxo which is bonded via a C atom;
or the salt of compound (1), especially a pharmaceutically acceptable salt thereof.

**12.** The use of a compound according to formula (7) in the synthesis of a compound according to fomula (2a) or (2).

**15.** The use of epichlorohydrin in the synthesis of compound according to formula (1) or (3).

**16.** Use according to claim 15, wherein the epichlorohydrin is (S)-epichlorohydrin.

**17.** A compound satisfying formula (8): 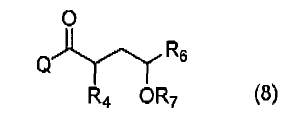 wherein R₄ is 2-propyl, and R₆, R₇, and Q have the meaning as described in claim 1.

**18.** A compound satisfying formula (10): 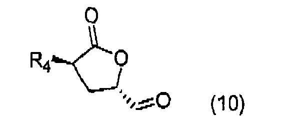 wherein R₄ is a branched C₃₋₆alkyl,
